(19) Europäisches Patentamt
European Patent Office
Office européen des brevets

(11) **EP 1 108 445 B1**

(12) **EUROPÄISCHE PATENTSCHRIFT**

(45) Veröffentlichungstag und Bekanntmachung des Hinweises auf die Patenterteilung:
**09.11.2005 Patentblatt 2005/45**

(51) Int Cl.$^7$: **A61N 1/365**

(21) Anmeldenummer: **00250431.4**

(22) Anmeldetag: **12.12.2000**

(54) **Herzschrittmacher mit Lagedetektor**

Cardiac pacemaker with position sensor

Stimulateur cardiaque avec capteur de position

(84) Benannte Vertragsstaaten:
**AT BE CH CY DE DK ES FI FR GB GR IE IT LI LU MC NL PT SE TR**

(30) Priorität: **17.12.1999 DE 19963245**

(43) Veröffentlichungstag der Anmeldung:
**20.06.2001 Patentblatt 2001/25**

(73) Patentinhaber: BIOTRONIK Mess- und Therapiegeräte GmbH & Co Ingenieurbüro Berlin
**12359 Berlin (DE)**

(72) Erfinder:
• **Czygan, Gerald**
  **91054 Buckenhof (DE)**
• **Lang, Martin**
  **91091 Grossenseebach (DE)**

(74) Vertreter: **Eisenführ, Speiser & Partner Patentanwälte Rechtsanwälte Spreepalais am Dom Anna-Louisa-Karsch-Strasse 2 10178 Berlin (DE)**

(56) Entgegenhaltungen:
**EP-A- 0 727 242    US-A- 5 628 777**

**Beschreibung**

Die Erfindung betrifft einen implantierbaren Herzschrittmacher mit einer Steuereinrichtung, die einen mit einem Bewegungssensor verbundenen Lagedetektor umfaßt.

Es ist bekannt, daß Lageänderungen bei Patienten mit Herzschrittmachern häufig unangemessene Reaktionen des Herzschrittmachers hervorrufen. Ein Grund dafür ist, daß Lagewechsel insbesondere wegen eines veränderten Gravitationseinflusses erhebliche Änderungen von Parametern des Blutkreislaufs hervorruft. Häufig wird eine durch Lagewechsel bedingte Änderung der interkardialen oder transthorakalen Impedanz von einer Steuerung des Herzschrittmachers als physische Aktivität mißdeutet, worauf der Herzschrittmacher mit einer Änderung der Herzfrequenz reagiert, die für den Lagewechsel unangemessenen ist. Dies führt zu Problemen. Zwar ist eine solche unangemessene Herzfrequenzänderung in der Regel nicht lebensbedrohend, dennoch aber für den Patienten spürbar. Insbesondere von einem liegenden Patienten, der lediglich seine Lage von rechtsseitig nach linksseitig ändert, werden dadurch hervorgerufene Herzfrequenzänderungen als unkomfortabel empfunden. Schwerwiegender ist, da bei selbstadaptierenden Herzschrittmachern durch solche überflüssigen Herzfrequenzänderungen das Adaptionsverhalten negativ beeinflußt werden kann, mit der Folge, daß der Herzschrittmacher auf physische Belastungen dann nicht mehr ausreichend reagiert.

Um dies zu vermeiden, werden Herzschrittmacher mit Detektoren für Lagewechsel versehen. Aus der US-A-5,593,431 ist ein Herzschrittmacher mit einem implantierten Akzelerometer als Lage-Detektor bekannt. Das dreiachsig wirkende Akzelerometer mißt die Richtung der Gravitation und bestimmt daraus die Lage des Patienten. Da jedoch Implantate generell dazu neigen, sich innerhalb des Körpers des Patienten zu drehen, besteht die Gefahr, daß sich das Bezugssystem des Akzelerometers verschiebt. Dadurch wird die Gravitationsmessung unzuverlässig und die Lageerkennung unsicher. Ferner ist es bekannt, Signale eines Akzelerometers einer Frequenzanalyse zu unterziehen, um daraus Ruhe- und Aktivitätsphasen zu bestimmen (US-A-5,233,984). Nachteilig an dieser Methode ist, daß ein Wechsel zwischen Ruhe- und Aktivitätsphase nicht unbedingt mit einem Lagewechsel korreliert. Dies führt zu Fehldetektionen, woraus eine nur geringe Erkennungsgenauigkeit resultiert.

Der Erfindung liegt die Aufgabe zugrunde, einen Herzschrittmacher zu schaffen, der eine zuverlässige und langzeitstabile Detektionseinrichtung für Lagewechsel aufweist.

Die erfindungsgemäße Lösung liegt in einem Herzschrittmacher mit den Merkmalen des Anspruchs 1. Vorteilhafte Weiterbildungen sind in den Unteransprüchen angegeben.

Erfindungsgemäß ist bei einem implantierbaren Herzschrittmacher mit einer Steuereinrichtung, die einen mit einem Bewegungssensor verbundenen Lagedetektor umfaßt, vorgesehen, daß der Lagedetektor eine Klassifikationseinrichtung zum Erkennen kurzer Bewegungen aufweist. Die Erfindung beruht auf dem Gedanken, daß ein kurzes Signal des Bewegungssensors ein Maßstab für einen Lagewechsel ist. Sie wendet sich damit von dem im Stand der Technik verfolgten Ansatz ab, Lagewechsel durch Interpretation von Inhalten der Signale des Bewegungssensors zu ermitteln. Dem liegt die Erkenntnis zugrunde, daß es entscheidend darauf ankommt, das Wechseln zwischen unterschiedlichen Lagen des Körpers zu ermitteln, und daß darauf verzichtet werden kann, die absolute Lage des Körpers des Patienten zu bestimmen. Die Erfindung hat erkannt, daß eine Klassifizierung nach der Zeitdauer des Signals des Bewegungssensors nicht nur zur zuverlässigen Detektion von Lagewechseln verwendet werden kann, sondern zusätzlich auch mit Vorteil zur Bestimmung der hämodynamischen Relevanz des Lagewechsels. Dem liegt die Erkenntnis zugrunde, daß ein Lagewechsel für die Hämodynamik des Blutkreislaufs um so bedeutender ist, je schneller er abläuft; andersherum ist ein langsam ablaufender Lagewechsel nur von geringer hämodynamischer Relevanz.

Der erfindungsgemäße Herzschrittmacher hat ferner den Vorteil, daß er nur wenig aufwendig ist. Bei dem Bewegungssensor kann es sich um einen einfachen bekannten Aktivitätssensor handeln, ein aufwendiges mehrachsiges Akzelerometer ist nicht erforderlich; auch Linearität und Empfindlichkeit sind nur von untergeordneter Bedeutung. Da der Bewegungssensor des erfindungsgemäßen Herzschrittmachers im Gegensatz zu dem dreiachsigen Akzelerometer kein bestimmtes Bezugssystem erfordert, ist der erfindungsgemäße Herzschrittmacher robust gegenüber Implantatdrehungen, wodurch sich die Langzeitzuverlässigkeit verbessert.

Um die Zeitdauer von einem Signal des Bewegungssensors bestimmen zu können, weist die Klassifikationseinrichtung zweckmäßigerweise einen Kurzzeit-Zähler und eine Schalteinrichtung auf. Der Begriff Zähler im Sinne der vorliegenden Erfindung ist weit zu verstehen; er umfaßt sowohl Ereigniszähler wie auch Zeitzähler. Zwar sind Zähler üblicherweise diskret arbeitend ausgebildet, jedoch sollen kontinuierlich arbeitende Zähler nicht ausgeschlossen sein. Von besonderem Vorteil ist ein Ereigniszähler, der zum Zählen von Takten ausgebildet ist, während ein Bewegungssignal anliegt. Da üblicherweise in Herzschrittmachern ein Taktsignal bereits für einen Mikroprozessor vorhanden ist, bleibt so der für den Zähler erforderliche Zusatzaufwand klein.

Zweckmäßigerweise ist die Vergleichseinrichtung so ausgebildet, daß sie am Ende des Bewegungssignals einen Zählerstand mit einem vorgebbaren Schwellwert vergleicht und ein Lageänderungssignal ausgibt. Mit dem vorgebbaren Schwellwert kann eine patientenindividuelle Einstellung der Zeitdauer erreicht werden, bis zu der ein Signal des Bewegungssensors als ein Lagewechsel detektiert werden soll. Auf diese Weise kann der erfindungsgemäße Herzschrittmacher an die Hämodynamik des jeweiligen Patienten angepaßt werden. Ggf. ist die Schalteinrichtung noch mit einer Telemetrieeinrichtung des Herzschrittmachers verbunden; dies ermöglicht eine Veränderung des Schwellwertes von außen auch nach einer Implantation. Die Schalteinrichtung stellt an ihrem Ausgang ein Lageänderungssignal bereit, das verschiedene Zustände annehmen kann und ein Eingangssignal für andere Funktionsmodule der Steuereinrichtung ist.

Vorteilhafterweise weist der Lagedetektor eine Gedächtniseinrichtung auf. In dieser sind vergangene Bewegungen gespeichert, wobei die Gedächtniseinrichtung nur eine beschränkte Erinnerungstiefe aufweist. Die Gedächtniseinrichtung stellt einen Indikator dafür dar, ob vor einer zu klassifizierenden Bewegung bereits andere Bewegungen stattgefunden haben, also ob ein Aktivitätszustand vorliegt, oder ob vorhergehend eine Ruhephase war, also ein Passivitätszustand vorliegt. Da ein Lagewechsel aus einem Passivitätszustand hämodynamisch relevanter ist als einer aus einem Aktivitätszustand, ist die Gedächtniseinrichtung von Vorteil zur Bewertung der hämodynamischen Relevanz eines Lagewechsels. Der Lagedetektor ist damit nicht auf gegenwärtig von den Sensoren gelieferte Signale beschränkt, sondern kann zusätzlich zur Auswertung von Signalen hinsichtlich vorhergegangener Bewegungen ausgebildet sein; dies ermöglicht eine genauere Erkennung von Lagewechseln. Die Gedächtniseinrichtung ist mit der Schalteinrichtung verbunden; die Schalteinrichtung ist so ausgebildet, daß sie entsprechend wie bei dem Kurzzeit-Zähler am Ende eines Bewegungssignal den Wert der Gedächtniseinrichtung mit einem vorgebbaren zweiten Schwellwert vergleicht. Mit dem vorgebbaren zweiten Schwellwert kann eine patientenindividuelle Einstellung der Gedächtniseinrichtung erreicht werden. Zweckmäßigerweise ist die Schalteinrichtung zum Verknüpfen der Vergleichsergebnisse des Kurzzeitzählers und der Gedächtniseinrichtung dergestalt ausgebildet, daß zum Ausgeben eines Lagewechselsignals das Bewegungssignal um so kürzer sein muß, je höher der Aktivitätszustand ist. Der Herzschrittmachers weist dann ein physiologisch richtiges Verhalten auf, wonach ein Lagewechsel um so eher eine entsprechende Reaktion des Herzschrittmachers erfordert, je weniger oder gar keine Aktivität vorausgegangen ist und umgekehrt.

Zweckmäßigerweise weist die Gedächtniseinrichtung einen Integrator auf. Der Integrator ist ausgebildet zum Addieren von Zeiträumen, während denen ein Signal des Bewegungssensors anliegt, und zum Subtrahieren von Zeiträumen, während denen kein Signal des Bewegungssensors anliegt. Somit wirkt er als ein Gedächtnis für vorhergegangene Bewegungen. Ein einstellbares Maximum für den Wert des Integrators begrenzt die Erinnerungstiefe.

Bei einer bevorzugten Ausführungsform sind der Kurzzeitzähler und der Integrator in einen Zähler integriert. Dies hat neben einem verringerten Aufwand den Vorteil, daß nur ein Schwellwert eingestellt werden braucht und dennoch die physiologisch richtige Wechselwirkung zwischen vorangegangener Aktivität und Zeitdauer des Bewegungssignals erhalten bleibt.

Zweckmäßigerweise ist ein Ablaufzähler vorgesehen, an dessen Eingang das Lagewechselsignal angelegt ist und an dessen Ausgang ein Sperrsignal ausgegeben wird. Ein solcher Zähler stellt ein Informationssignal darüber zur Verfügung, wie lange bereits das Lageänderungssignal anliegt und ermöglicht somit eine Bestimmung der Zeit, die seit dem detektierten Lagewechsel verstrichen ist. Dies ermöglicht es der Steuereinrichtung, zeitlich abgestimmt auf einen Lagewechsel zu reagieren, bspw. ihre Reaktion auf einen bestimmten Zeitraum zu begrenzen.

Zweckmäßigerweise ist eine Löscheinrichtung für das Sperrsignal vorgesehen, deren Auslöser mit dem Bewegungssensor verbunden ist. Durch die Löscheinrichtung wird erreicht, daß beim Auftreten einer neuerlichen Bewegung das Lagewechselsignal gelöscht wird und der Herzschrittmacher unbeeinträchtigt durch das Lagewechselsignal auf die neuerliche Bewegung reagieren kann.

Vorzugsweise weist die Steuereinrichtung einen lageabhängigen Herzratenbegrenzer auf. Damit kann erreicht werden, daß die Stimulationsfrequenz bei lagewechselbedingten Bewegungen des Patienten nicht über einen bestimmten Grenzwert steigt. Zweckmäßigerweise sind an den Herzratenbegrenzer ein Lageänderungssignal und ein Herzratensignal angelegt. Bei dem Herzratensignal handelt es sich um ein von der Steuereinrichtung berechnetes Signal zur Erregung des Herzmuskels. Abhängig von dem Lageänderungssignal begrenzt der Herzratenbegrenzer das berechnete Herzratensignal mittels einer einstellbaren Begrenzungsfunktion und gibt es aus. Unter einer Begrenzungsfunktion wird eine Funktion verstanden, deren Ausgangswert geringer ansteigt als ihr Eingangswert. Der Herzratenbegrenzer kann so ausgebildet sein, daß bei einem Wert des berechneten Herzratensignals, der größer ist als ein Grenzwert, das ausgegebene Herzratensignal genau diesen Grenzwert aufweist; dies wird als starre Begrenzung bezeichnet. Vorteilhafterweise ist der Herzratenbegrenzer jedoch weich begrenzend ausgebildet, daß bei einem Wert der berechneten Herzraten, der größer ist als ein Grenzwert, der Wert der ausgegebenen Herzrate zwischen der berechneten Herzrate und dem Grenzwert liegt.

Mit Vorteil weist die Steuereinrichtung einen Inhibitor auf. Der Inhibitor dient dazu, eine durch Lagewechsel induzierte Adaption von Parametern der Steuereinrichtung zu verhindern. Damit wird vermieden, daß die Parameter durch Lagewechsel so verändert werden, daß das frequenzadaptive Verhalten des Herzschrittmachers verschlechtert wird. Dazu ist zweckmäßigerweise vorgesehen, daß der Inhibitor mit einem Adaptionsmodul derart zusammenwirkend verbunden ist, daß eine Adaption lageabhängig gesperrt ist. Dadurch wird vermieden, daß selbstadaptierende Parameter des Herzschrittmachers während des Anliegens des Lagewechselsignal beeinflußt werden, andernfalls könnte das Verhalten des Herzschrittmachers bei physischer Belastung verschlechtert werden.

In einer alternativen Ausführungsform ist der implantierbare Herzschrittmacher mit einem Sensor für den physiologischen Bedarf, insbesondere einem Impedanzsensor zum Messen der-intrakardialen odertransthorkalen Impedanz verbunden. Außerdem umfaßt dieser Herzschrittmacher Steuermittel zur Einstellung einer Stimulations- oder Herzrate in Abhängigkeit eines von dem Sensor für den physiologischen Bedarf stammenden Signals. Die Steuerung der Herzrate ist dabei derart begrenzbar, daß die Ratenänderungen infolge des physiologischen Bedarfs in ihrer Höhe insbesondere nach oben, aber auch nach unten begrenzt sind. Es ergibt sich somit ein Band zugelassener Herzraten. Bei dem alternativen Herzschrittmacher wird die Lage dieses Bandes, daß heißt die Lage der oberen und unteren Ratenbegrenzung durch ein von dem Bewegungssensor stammendes Signal bestimmt. Auf diese Weise läßt sich eine Ratenbegrenzung im Falle eines Lagewechsels ebenso erzielen, wie eine erhöhte Stimulationsrate bei andauernder Bewegung und erhöhtem metabolischen Bedarf zugelassen werden kann.

Die Erfindung wird nachfolgend unter Bezugnahme auf die beigefügte Zeichnung, die bevorzugte Ausführungsbeispiele der Erfindung darstellt, näher erläutert. Darin zeigen

- Figur 1 einen erfindungsgemäßen Herzschrittmacher mit einem Bewegungssensor und einer Steuereinrichtung, die einen Lagedetektor aufweist, in einer schematisierten Ansicht;

- Figur 2 ein Hochsprachenlisting zum Einspeichern bei einem zweiten Ausführungsbeispiel der Erfindung;

- Figur 3 Schaubilder einiger Signale bei einem Ausführungsbeispiel der Erfindung;

- Figur 4 weitere Schaubilder einiger Signale; und

- Figur 5 einen erfindungsgemäßen Herzschrittmacher gemäß einem zweiten Ausführungsbeispiel der Erfindung.

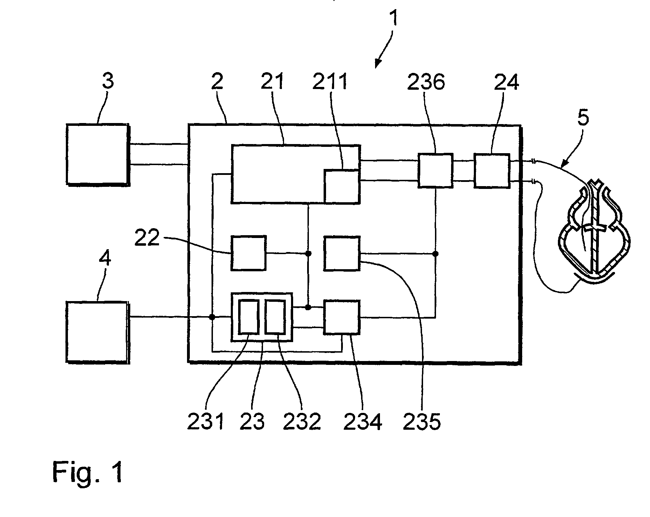

In Fig. 1 ist ein Herzschrittmacher nach einem Ausführungsbeispiel der Erfindung dargestellt. Der Herzschrittmacher 1 umfaßt in einem nicht dargestellten Gehäuse eine Steuereinrichtung 2, die mit einer Energieversorgung 3 und einem Bewegungssensor 4 verbunden ist. Ferner ist ein an zur Anordnung an einem Herz vorgesehener Elektrodensatz 5 symbolhaft dargestellt, der mit dem Herzschrittmacher 1 verbunden ist.

Der Bewegungssensor 4 ist als ein einfacher Aktivitätssensor ausgeführt, wie er einem Fachmann aus dem Stand der Technik bekannt ist. Beispiele für solche Aktivitätssensoren sind Beschleunigungsgeber und Vibrationsgeber. Da der Bewegungssensor 4 lediglich das Vorhandensein oder Fehlen einer Bewegung bestimmen soll, kann er verhältnismäßig einfach und robust aufgebaut sein. Da ein richtungsunabhängiges Bestimmen ausreicht, kommt es auf die genaue Lage des Bewegungssensors 4 im implantierten Zustand und darauf, ob sie auch langzeitstabil beibehalten wird, nicht entscheidend an. Vorzugsweise ist der Bewegungssensor in das Gehäuse des Herzschrittmachers 1 integriert angeordnet.

Die Steuereinrichtung 2 weist als Kern eine Herzerregungseinheit 21 auf, und ist mit der Energieversorgung 3 und über eine Leistungsendstufe 24 mit dem Elektrodensatz 5 verbunden. Die Herzerregungseinheit 21 ist dazu ausgebildet, aus Signalen von dem Bewegungssensor 4 und/oder weiteren, nicht dargestellten Sensoren eine Herzrate zu berechnen, die als Impulsfolge an den Elektrodensatz 5 ausgegeben wird. Sie umfaßt ein Adaptionsmodul 211, das zur selbsttätigen Adaption von Parametern, die bei der Berechnung der Herzrate verwendet werden, ausgebildet ist. Solch eine Herzerregungseinheit 21 mit Adaptionsmodul 211 ist dem Fachmann aus dem Stand der Technik bekannt und wird daher nachfolgend nicht näher beschrieben.

Die Steuereinrichtung 2 weist ferner einen Taktgeber 22, eine Klassifikationseinrichtung 23, einen Ablaufzähler 234, einen Inhibitor 235, und einen Limiter 236 auf. Diese sind soweit erforderlich mittels nicht dargestellter Verbindungen mit der Energieversorgung 3 verbunden.

Der Taktgeber 22 ist als ein Zyklusgeber ausgeführt, der für jeden Herzschlag einen Taktpuls ausgibt. Dies bedeutet, daß die Taktpulse nicht mit einer konstanten Frequenz ausgegeben werden, sondern daß die Frequenz der Taktpulse von der aktuellen Herzrate abhängt. Es soll aber nicht ausgeschlossen sein, daß ein konventioneller Uhrenbaustein als Taktgeber vorgesehen ist. Der Taktgeber stellt die Zeitbasis für den Herzschrittmacher 1 bereit. Sein Ausgang ist außer mit der Herzerregungseinheit 21 mit der Klassifikationseinrichtung 23 und dem Ablaufzähler 234 verbunden.

Die Klassifikationseinrichtung 23 umfaßt einen Zähler 231 und eine Schalteinheit 232. Der Zähler 231 ist als ein maximumbegrenzter Auf-/Abwärtszähler mit einem Zähleingang und einem Steuereingang sowie einem Zählerstandausgang ausgeführt. Mit seinem Zähleingang ist der Taktgeber 22 verbunden, mit seinem die Zählrichtung bestimmenden Steuereingang der Bewegungssensor 4. Ferner weist der Zähler 231 ein einstellbares Maximum auf, dessen Wert von dem Zählerstand nicht überschritten werden kann; außerdem kann der Wert Null nicht unterschritten werden. Liegt ein Signal des Bewegungssensors 4 an dem Steuereingang des Zählers 231 an, ist der Zähler 231 als Aufwärtszähler geschaltet; die an dem Zähleingang anliegenden Taktsignale des Taktgebers werden zu dem Zählerstand dazugezählt. Liegt kein Signal des Bewegungssensors 4 an dem Steuereingang an, ist der Zähler 231 als Abwärtszähler geschaltet; die an dem Zähleingang anliegenden Taktsignale des Taktgebers vermindern den Zählerstand.

An einen Eingang der Schalteinheit 232 ist der Ausgang des Zählers 231 angelegt. Die Schalteinheit 232 ist so ausgebildet, daß sie am Ende einer Bewegung, also sowie das Signal vom Bewegungssensor 4 ausbleibt, den Zählerstand des Zählers 231 mit einem einstellbaren Schwellwert vergleicht und dann, wenn der Zählerstand kleiner als der Schwellwert ist, ein Lagewechselsignal ausgibt. Um zu erkennen, wann das Signal des Bewegungssensors 4 endet, ist ein weiterer Eingang der Schalteinheit 232 vorgesehen, an den der Bewegungssensor 4 angelegt ist; das Ausbleiben des Signals von dem Bewegungssensor 4 löst in der Schalteinheit 232 das Vergleichen des Zählerstands mit dem Schwellwert aus. Alternativ kann auch vorgesehen sein, den Beginn einer Verminderung des Zählerstands als das Ende des Signals des Bewegungssensors 4 anzunehmen; zwar geht damit eine Verzögerung um einen Taktimpuls gegenüber dem wahren Ende des Signals des Bewegungssensors einher, jedoch erspart dies den Aufwand eines zusätzlichen Eingangs.

Das Lagewechselsignal ist an einen Eingang des Ablaufzählers 234 angelegt und bewirkt, daß der Ablaufzähler 234 auf einen voreinstellbaren Verzögerungswert eingestellt wird und von diesem ausgehend abwärts bis Null zählt. Während des Ablaufens gibt der Ablaufzähler 234 ein Sperrsignal aus. Ein Ausgang des Ablaufzählers 234 ist mit dem Inhibitor 235 und dem Limiter 236 verbunden. An diese wird das Sperrsignal angelegt. Ferner weist der Ablaufzähler einen Löscheingang auf, an den der Bewegungssensor 4 angelegt ist. Tritt während des Ablaufens des Ablaufzählers 234 ein Signal des Bewegungssensors 4 auf, so wird der Ablaufzähler 234 gelöscht und die Ausgabe des Sperrsignals beendet.

Der Inhibitor 235 weist einen Eingang, an den das Sperrsignal angelegt ist, und einen Ausgang auf, der mit dem Adaptionsmodul 211 der Herzerregungseinheit 21 verbunden ist. Der Inhibitor 235 ist in einem aktiven Zustand, solange das Sperrsignal an seinem Eingang anliegt; andernfalls ist er in einem passiven Zustand. Er wirkt derart mit dem Adaptionsmodul 211 zusammen, daß er in seinem aktiven Zustand das Adaptionsmodul 211 sperrt. Dadurch, daß die Parameteradaption gesperrt ist, wird insbesondere verhindert, daß die Lagewechsel zu einer nicht gewünschten Veränderung eines Antwortfaktor-Parameters ("response factor") führen.

Der Limiter 236 ist zwischen der Herzerregungseinrichtung 21 und der Leistungsendstufe 24 angeordnet. Dazu ist er mit einem Signaleingang mit dem Ausgang der Herzerregungseinrichtung 21 und mit einem Signalausgang mit dem Eingang der Leistungsendstufe 24 verbunden. Ferner weist er einen Steuereingang auf, an den das Sperrsignal angelegt ist. Der Limiter 236 ist in einem aktiven Zustand, solange das Sperrsignal an seinem Steuereingang anliegt; andernfalls ist er in einem passiven Zustand. In seinem aktiven Zustand begrenzt er die Frequenz der an dem Signaleingang anliegenden Herzrate, soweit sie größer ist als eine einstellbare Schwellfrequenz ("ThresholdRate"). Die Begrenzung der Herzrate erfolgt bei dem in dem Ausführungsbeispiel dargestellten Limiter 236 "weich", d. h. die Frequenz der an die Leistungsstufe 24 ausgegebenen Herzrate (HRout) liegt um einen geringeren Betrag über der Schwellfrequenz als die Frequenz der von der Herzerregungseinrichtung 21 angelegten Herzrate (HRin). Genauer gesagt ist die ausgegebenen Herzrate im Begrenzungsfall bestimmt durch die Formel:

$$HRout = (HRin - ThresholdRate)/8 + ThresholdRate.$$

Damit bleibt auch im Begrenzungsfall, d. h. wenn das Sperrsignal am Limiter 236 anliegt und die angelegte Herzrate über dem Schwellwert ist, das Verhalten des Herzschrittmachers 1 physiologisch richtig, nämlich zumindest schwach positiv chronotrop. Ferner ist der Limiter 236 so ausgebildet, daß die ausgegebene Frequenz auf einen absoluten Höchstwert ("MCLR") begrenzt ist. Der Limiter 236 ist rückwirkungsfrei ausgebildet, so daß auch dann, wenn die ausgegebene Herzrate über dem absoluten Höchstwert liegen würde, eine Verringerung des Response-Faktors unterbunden ist. Dadurch wird auch bei extremen Lagewechseln, die zu heftiger Reaktion des Herzschrittmachers führt, eine negative Beeinflussung von Parametern des Herzschrittmachers vermieden.

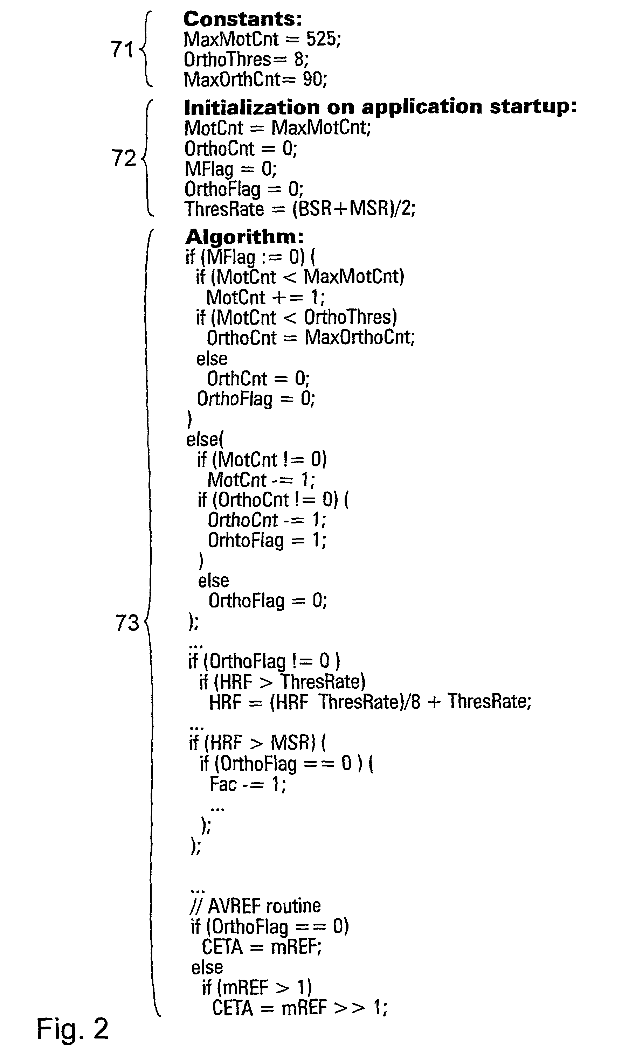

Die vorgenannten Bestandteile der Steuereinrichtung 2 brauchen nicht unbedingt als eigenständige Bauteile in Hardware realisiert sein; sie können auch als Module der Steuereinrichtung 2 ausgeführt sein, wobei dann die Steuereinrichtung einen Mikrokontroller und einen Speicher aufweist, in dem ein Programmcode gespeichert ist. Ein Ausführungsbeispiel für einen solchen in dem Speicher gespeicherten Programmcode ist in Fig. 2 dargestellt.

In einem ersten Abschnitt 71 sind Konstanten definiert. Hierbei handelt es sich um die maximale Grenze ("MaxMotCnt") des Zählers, den Schwellwert ("OrthoThres") der Schalteinheit und den Verzögerungswert ("MaxOrthCnt") des Ablaufzählers.

In einem zweiten Abschnitt 72 sind Variablen und Indikatoren initialisiert. Der Wert des Zählers ("MotCnt") ist auf die maximale Grenze gesetzt, der Ablaufzähler ("OrthoCnt") ist auf Null gesetzt, Indikatoren für Bewegung ("MFlag") und Lagewechsel ("OrthoFlag") sind auf Null gesetzt und die Schwellfrequenz ("ThresRate") ist bestimmt.

In einem dritten Abschnitt 73 sind die Module der Steuereinrichtung bestimmt. Dazu wird in einem ersten Schritt geprüft, ob ein Bewegungssignal anliegt. Ist das der Fall, so wird der Wert des Zählers um eins erhöht, sofern noch nicht die maximale Grenze erreicht ist; der Ablaufzähler wird vorsorglich, falls der Wert des Zählers kleiner als der Schwellwert ist, auf den Verzögerungswert gesetzt, andernfalls auf Null gesetzt; außerdem wird der Lagewechselindikator auf Null gesetzt. Liegt jedoch der Fall vor, daß ein Bewegungssignal nicht anliegt, so wird der Wert des Zählers um Eins vermindert, sofern er noch nicht Null erreicht hat; außerdem wird, falls der Ablaufzähler einen von Null verschiedenen Wert aufweist, der Wert des Ablaufzählers um eins vermindert und der Indikator für Lagewechsel gesetzt, andernfalls wird nur der Indikator für Lagewechsel gelöscht. In einem nachfolgenden zweiten Schritt wird geprüft, ob die von der Herzerregungseinrichtung 21 ausgegebene Herzrate größer als die Schwellfrequenz ist. Sofern das der Fall ist, wird eine verminderte Herzrate berechnet, indem eine Differenz zwischen der Herzrate und der Schwellfrequenz gebildet, durch acht geteilt und zu der Schwellfrequenz addiert wird.

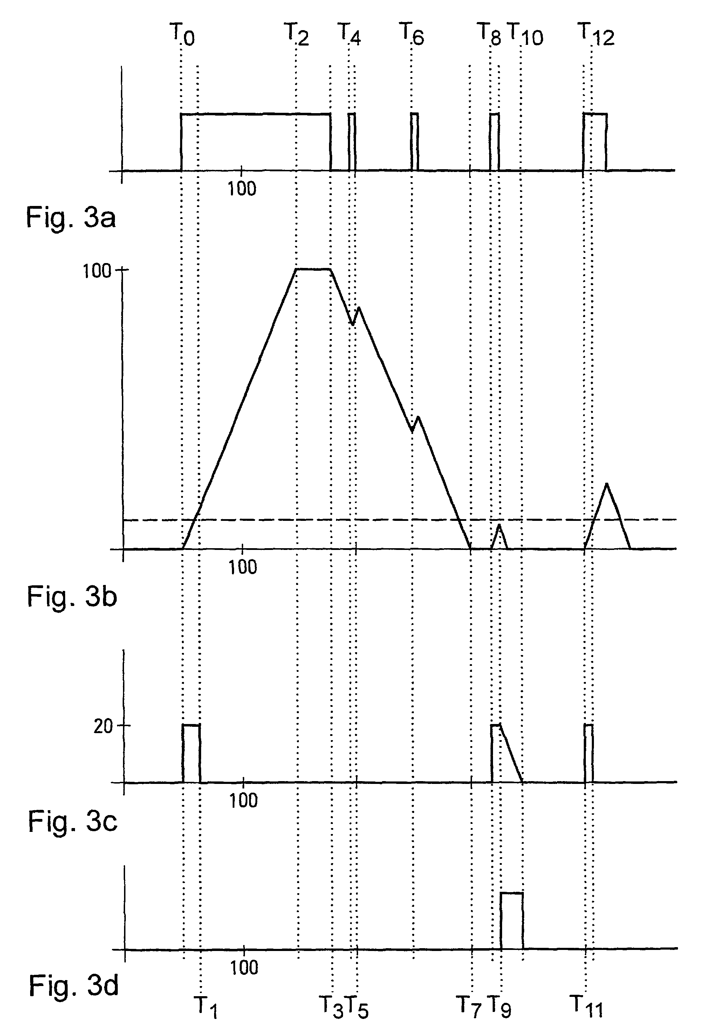

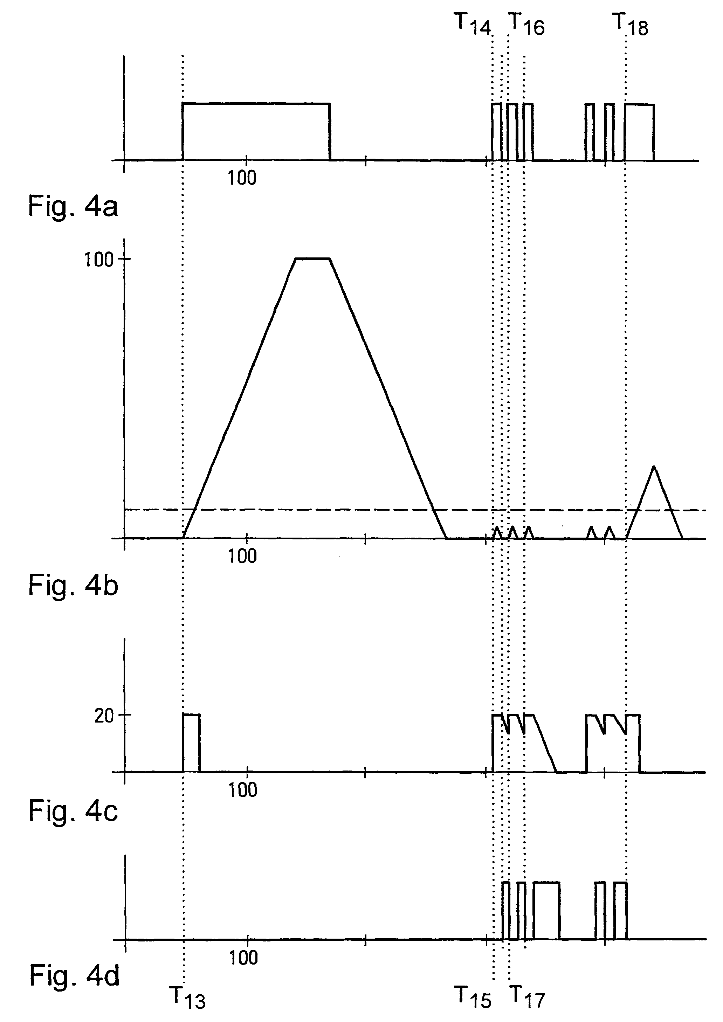

Der Betrieb des erfindungsgemäßen Herzschrittmachers bei der Lagedetektion wird nachfolgend anhand zweier in den Fig. 3 und Fig. 4 dargestellter Beispiele erläutert.

In Fig. 3 ist bei a) ein Signalverlauf des Bewegungssensors 4 dargestellt. Es sind eine längere Bewegungsphase am Anfang und mehrere kürzere nachfolgende Bewegungsphasen zu erkennen. Bei b) ist zeitlich zugeordnet der Zählerstand des Zählers 231 dargestellt; ferner ist der Schwellwert mittels einer gestrichelten horizontalen Linie dargestellt. Bei c) ist der Wert des Ablaufzählers 234 dargestellt. Bei d) ist das Lagewechselsignal dargestellt. Auf der Abszisse sind Pulse des Taktgebers dargestellt.

Es ist zu erkennen, daß mit Beginn der ersten Bewegungsphase zu einem Zeitpunkt T0 der Zähler 231 von Null beginnend hochzählt; gleichzeitig wird der Ablaufzähler 234 vorsorglich mit dem Verzögerungswert geladen. Ein Lagewechselsignal wird nicht ausgegeben. Im weiteren Verlauf der ersten Bewegungsphase erhöht sich der Zählerstand b) des Zählers und überschreitet zum Zeitpunkt T1 den durch die gestrichelte horizontale Linie dargestellten Schwellwert. Zu diesem Zeitpunkt T1 hat die Bewegungsphase eine Dauer erreicht, die zu lang ist, als daß es sich um einen Lagewechsel handeln könnte; am Schluß dieser Bewegungsphase wird kein Lagewechselsignal ausgegeben. Die erfindungsgemäße Klassifikationseinrichtung löscht daher den vorsorglich bereits geladenen Ablaufzähler 234 indem sie ihn auf Null setzt. Zu einem Zeitpunkt T2 erreicht der Zähler 231 sein Maximum, der Zählerstand 231 verharrt für die restliche Dauer der ersten Bewegungsphase auf diesem Wert. Mit Ende der ersten Bewegungsphase zum Zeitpunkt T3 beginnt sich der Zählerstand a) des Zählers 231 zu vermindern. Ein Lagewechselsignal wird nicht ausgegeben. Zu dem Zeitpunkt T4 beginnt eine zweite Bewegungsphase, die kürzer als die erste ist. Der Zähler 231 zählt aufwärts und der Zählerstand a) erhöht sich, bis die zweite Bewegungsphase zu einem Zeitpunkt T5 endet. Obwohl die zweite Bewegungsphase kurz genug ist, um ein Lagewechsel sein zu können, gibt die Klassifikationseinrichtung kein Lagewechselsignal aus, da der Zählerstand a) des Zählers 231 größer als der Schwellwert (horizontale Linie) ist. Der Zähler 231 wirkt hierbei als Gedächtnis der Klassifikationseinrichtung, da er sozusagen noch weiß, daß kurz zuvor noch eine Bewegung stattgefunden hat und damit eine besondere Reaktion auf einen eventuellen Lagewechsel nicht erforderlich ist. Zu einem Zeitpunkt T6 beginnt eine dritte Bewegungsphase, die der zweiten Bewegungsphase entspricht. Nach dieser Bewegungsphase zählt der Zähler 231 weiter abwärts, bis sein Zählerstand a) zu einem Zeitpunkt T7 den Wert Null erreicht. Zu einem Zeitpunkt T8 beginnt eine vierte Bewegungsphase. Der Zähler 231 zählt hoch, der Zählerstand a) erhöht sich und der Ablaufzähler 234 wird vorsorglich mit dem Verzögerungswert c) geladen. Zu einem Zeitpunkt T9 endet die vierte Bewegungsphase, wobei der Zählerstand a) unterhalb des Schwellwerts (horizontale Linie) liegt. Die Klassifikationseinrichtung startet daher den Ablaufzähler 234. Dieser zählt runter und dabei wird das Lagewechselsignal d) ausgegeben, bis zu einem Zeitpunkt T10 der Ablaufzähler 234 den Wert Null erreicht und das Ausgeben des Lagewechselsignals d) endet. Zu einem Zeitpunkt T11 beginnt eine fünfte Bewegungsphase. Der Zähler 231 zählt aufwärts und der Ablaufzähler 234 wird vorsorglich mit dem Verzögerungswert geladen. Zu einem Zeitpunkt T12 überschreitet der Zählerstand b) den Schwellwert, d. h. die Bewegungsphase ist zu lang, um ein Lagewechsel zu sein. Der Ablaufzähler 234 wird daher auf Null gesetzt, es wird kein Lagewechselsignal am Ende der fünften Bewegungsphase ausgegeben.

In Fig. 4 ist ein weiteres Funktionsbeispiel für den erfindungsgemäßen Herzschrittmacher dargestellt. Wie bei Fig. 3 ist bei a) ein Signalverlauf des Bewegungssensors 4, bei b) Zählerstände des Zählers 231, bei c) Werte des Ablaufzählers 234 und bei d) ein Lagewechselsignal dargestellt. Auf der Abszisse sind Pulse des Taktgebers dargestellt.

Die zu einem Zeitpunkt T13 beginnende sechste Bewegungsphase entspricht weitgehend der ersten und wird nachfolgend nicht weiter erläutert. Zu einem Zeitpunkt T14 beginnt eine siebte Bewegungsphase, die mehrere Einzel-Bewegungen umfaßt. Mit Beginn der Bewegungsphase zum Zeitpunkt T14 erhöht sich der Zählerstand b) des Zählers 231, vorsorglich wird der Ablaufzähler 234 auf den Verzögerungswert c) gesetzt. Am Ende der ersten Einzel-Bewegung zu einem Zeitpunkt T15 liegt der Zählerstand a) unter dem Schwellwert (horizontale gestrichelte Linie) und die Klassifikationseinrichtung startet den Ablaufzähler 234 und gibt das Lagewechselsignal d) aus. Mit Beginn der zweiten Einzel-Bewegung zu einem Zeitpunkt T16 erhöht sich wieder der Zählerstand b) des Zählers 231, der Ablaufzähler wird vorsorglich auf den Verzögerungswert c) gesetzt und das Lagewechselsignal d) wird gelöscht. Durch letzteres ist sichergestellt, daß der Herzschrittmacher ohne eventuelle Beeinträchtigungen durch das Lagewechselsignal in geeigneter Weise auf die aktuelle Bewegung reagieren kann. Handelt es sich bei der folgenden, zu einem Zeitpunkt T 17 beginnenden Einzel-Bewegung um eine kurze Bewegung (wie dargestellt), so liegt am Ende dieser Einzel-Bewegung zu einem Zeitpunkt T18 der Zählerstand a) unter dem Schwellwert; daraufhin wird der Ablaufzähler 234 gestartet und das Lagewechselsignal d) ausgegeben. Handelt es sich jedoch bei einer nachfolgenden Bewegung um eine längerdauernde Bewegung (wie die zu einem Zeitpunkt T19 beginnende), so wächst der Zählerstand b) über den Schwellwert hinaus an und es wird kein Lagewechselsignal mehr ausgegeben.

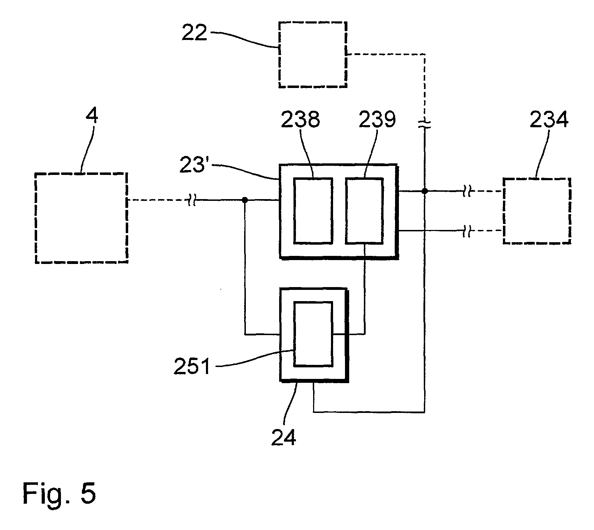

In Fig. 5 ist ein drittes Ausführungsbeispiel eines erfindungsgemäßen Herzschrittmachers dargestellt. Sie weist in Gestalt eines Integrators 251 eine eigenständige Gedächtniseinrichtung 25 auf, während die Gedächtniseinrichtung bei dem in Fig. 1 dargestellten ersten Ausführungsbeispiel in den Zähler 231 integriert ist. Die Klassifikationseinrichtung 23' umfaßt einen Kurzzeitzähler 238 und eine Schalteinheit 239.

Der Integrator 251 ist als verlustbehafteter Integrierer ausgebildet, d. h. er erhöht seinen Wert, wenn ein Signal von dem Bewegungssensor 4 anliegt, und er vermindert seinen Wert, wenn kein Signal von dem Bewegungssensor 4 anliegt. Dazu ist er als ein maximumbegrenzter Auf-/Abwärtszähler mit einem Zähleingang und einem Steuereingang sowie einem Summenwertausgang ausgeführt. Der Integrator 251 braucht jedoch nicht unbedingt als ein Zähler ausgeführt sein, es kann sich auch um ein anderes Bauelement mit integrierender Funktion, vorzugsweise tiefpaßartiges Element wie beispielsweise ein RC-Element, handeln. Mit einem Zähleingang des Integrators 251 ist der Taktgeber 22 verbunden, mit seinem die Zählrichtung bestimmenden Steuereingang der Bewegungssensor 4. Ferner weist der Integrator 251 ein einstellbares Maximum auf, dessen Wert von dem Zählerstand nicht überschritten werden kann; außerdem kann der Wert Null nicht unterschritten werden. Während das Signal von dem Bewegungssensor 4 anliegt, summiert der Integrator 251 die Taktpulse des Taktgebers 22 auf. Während kein Signal von dem Bewegungssensor 4 anliegt, vermindert der Integrator 251 mit jedem Taktpuls seinen Wert um eins. Der Wert des Integrators 251 stellt ein Maß für den Umfang der Bewegungen dar, die von dem Bewegungssensor 4 in einer unmittelbaren Vergangenheit detektiert wurden. Dabei ist nur ein gewisser Bereich der jüngeren Vergangenheit relevant, der durch die Höhe des einstellbaren Maximums bestimmt ist. Der Integrator 251 wirkt somit als ein seiner Erinnerungstiefe beschränktes Gedächtnis des Herzschrittmachers.

Der Kurzzeitzähler 238 ist als ein rücksetzbarer Ereigniszähler ausgeführt. Er weist einen Zähleingang, an den der Taktgeber 22 angelegt ist, einen Schalteingang, an den der Bewegungssensor 4 angelegt ist, und einen Zählstandausgang auf, mit dem die Schalteinheit 239 verbunden ist. Der Schalteingang ist so ausgeführt, daß flankengesteuert. Der Kurzzeitzähler 238 ist so ausgebildet, daß sein Zählerstand beim Ansteigen eines Signals des Bewegungssensors 4 am Schalteingang auf Null zurückgesetzt wird, und daß er die Anzahl der Taktpulse des Taktgebers 22 zählt, während ein Signal von dem Bewegungssensor 4 an dem Schalteingang angelegt ist.

Die Schalteinheit 239 weist Eingänge auf, die mit dem Integrator 251 bzw. dem Kurzzeitzähler 238 verbunden sind, und einen Ausgang auf, der zum Ausgeben eines Lagewechselsignals ausgebildet ist. Die Schalteinheit 239 ist so ausgebildet, daß sie am Ende einer Bewegung, also mit dem Ausbleiben des Signals des Bewegungssensors 4, den an seinem ersten Eingang anliegenden Summenwert des Integrators 251 mit einer einstellbaren Schwelle vergleicht, und ferner den an ihrem zweiten Eingang anliegenden Zählerstand des Kurzzeitzählers miteiner einstellbaren Schwelldauer vergleicht; nur wenn beide Eingänge unter der Schwelle bzw. Schwelldauer liegen, wird über den Ausgang der Schalteinheit 239 das Lagewechselsignal ausgegeben. Damit zu einer Bewegung das Lagewechselsignal ausgegeben werden kann, müssen also zwei Bedingungen kumulativ erfüllt sein: die zu bewertende Bewegung muß kurz genug gewesen sein, nämlich kürzer als die Schwelldauer; und es dürfen nicht zu viele Bewegungen in der jüngeren Vergangenheit stattgefunden haben, nämlich weniger als durch die Schwelle des Integrators bestimmt. Beide Bedingungen können unabhängig voneinander durch Verändern der Schwelle bzw. Schwelldauer an individuelle Gegebenheiten eines Patienten angepaßt werden.

## Patentansprüche

1. Implantierbarer Herzschrittmacher mit einer Steuereinrichtung (2), die einen Lagedetektor umfaßt, der mit einem Bewegungssensor (4) verbunden ist, **dadurch gekennzeichnet, daß** der Lagedetektor eine Klassifikationseinrichtung (23) zum Erkennen kurzzeitiger Bewegungen aufweist.

2. Implantierbarer Herzschrittmacher nach Anspruch 1, **dadurch gekennzeichnet, daß** die Klassifikationseinrichtung (23) einen Kurzzeitzähler (238) und eine Schalteinrichtung (239) aufweist.

**3.** Implantierbarer Herzschrittmacher nach Anspruch 1 oder 2, **dadurch gekennzeichnet, daß** der Lagedetektor eine Gedächtniseinrichtung (25) aufweist.

**4.** Implantierbarer Herzschrittmacher nach Anspruch 3, **dadurch gekennzeichnet, daß** die Gedächtniseinrichtung (25) einen Integrator (251) aufweist.

**5.** Implantierbarer Herzschrittmacher nach Anspruch 4, **dadurch gekennzeichnet, daß** Kurzzeitzähler (238) und Integrator (251) in einen Zähler (231) integriert sind.

**6.** Implantierbarer Herzschrittmacher nach einem der Ansprüche 2 bis 5, **dadurch gekennzeichnet, daß** die Schalteinrichtung (232) so ausgebildet ist, daß sie am Ende des Bewegungssignals einen Zählerstand mit mindestens einem vorgebbaren Schwellwert vergleicht und ein Lagewechselsignal ausgibt.

**7.** Implantierbarer Herzschrittmacher nach einem der Ansprüche 1 bis 6, **dadurch gekennzeichnet, daß** ein Ablaufzähler (234) vorgesehen ist, an dessen Eingang das Lagewechselsignal angelegt ist und an dessen Ausgang ein Sperrsignal ausgegeben wird.

**8.** Implantierbarer Herzschrittmacher nach Anspruch 7, **dadurch gekennzeichnet, daß** eine Löscheinrichtung für das Sperrsignal vorgesehen ist, deren Auslöser mit dem Bewegungssensor (4) verbunden ist.

**9.** Implantierbarer Herzschrittmacher nach einem der Ansprüche 1 bis 8, **dadurch gekennzeichnet, daß** die Steuereinrichtung einen lageabhängigen Herzratenbegrenzer (236) aufweist.

**10.** Implantierbarer Herzschrittmacher nach Anspruch 9, **dadurch gekennzeichnet, daß** an den Herzratenbegrenzer (236) das Lageänderungssignal und ein Herzratensignal angelegt sind.

**11.** Implantierbarer Herzschrittmacher nach einem der Ansprüche 1 bis 10, **dadurch gekennzeichnet, daß** die Steuereinrichtung einen Inhibitor (235) aufweist.

**12.** Implantierbarer Herzschrittmacher nach Anspruch 11, **dadurch gekennzeichnet, daß** der Inhibitor (235) mit einem Adaptionsmodul (211) derart zusammenwirkend verbunden ist, daß eine Adaption lageabhängig gesperrt ist.

**13.** Implantierbarer Herzschrittmacher nach einem der Ansprüche 1 bis 12, **dadurch gekennzeichnet, daß** der Herzschrittmacher mit einem Sensor für den physiologischen Bedarf, insbesondere einem Impedanzsensor zum Messen der intrakardialen oder transthorakalen Impedanz verbunden ist und Steuermittel zur Einstellung einer Stimulations- oder Herzrate in Abhängigkeit eines vom Sensor für den physiologischen Bedarf stammenden Signals umfaßt, wobei die Änderung der Stimulations- oder Herzrate dauerhaft derart begrenzbar ist, daß die Herzratenbegrenzung in Abhängigkeit von einem Ausgangssignal des Begrenzungssensor (4) erfolgt.

**Claims**

**1.** Implantable cardiac pacemaker comprising a control device (2) including a position detector connected to a movement sensor (4), **characterised in that** the position detector comprises a classification device (23) for recognising momentary movements.

**2.** Implantable cardiac pacemaker according to claim 1, **characterised in that** the classification device (23) comprises a short-term counter (238) and a switch device (239).

**3.** Implantable cardiac pacemaker according to either claim 1 or claim 2, **characterised in that** the position detector comprises a memory device (25).

**4.** Implantable cardiac pacemaker according to claim 3, **characterised in that** the memory device (25) comprises an integrator (251).

**5.** Implantable cardiac pacemaker according to claim 4, **characterised in that** the short-term counter (238) and the integrator (251) are integrated into a counter (231).

**6.** Implantable cardiac pacemaker according to any one of claims 2 to 5, **characterised in that** the switch device (232) is such that at the end of the movement signal it compares a counter reading to at least one predeterminable threshold value and issues a position change signal.

**7.** Implantable cardiac pacemaker according to any one of claims 1 to 6, **characterised in that** there is provided an elapsed time counter (234), to the input of which the position change signal is applied and at the output of which a blocking signal is issued.

**8.** Implantable cardiac pacemaker according to claim 7, **characterised in that** there is provided a clearing device for the blocking signal, the triggering device of which is connected to the movement sensor (4).

**9.** Implantable cardiac pacemaker according to any one of claims 1 to 8, **characterised in that** the control device has a position-dependent heart rate limiter (236).

10. Implantable cardiac pacemaker according to claim 9, **characterised in that** the position change signal and a heart rate signal are applied to the heart rate limiter (236).

11. Implantable cardiac pacemaker according to any one of claims 1 to 10, **characterised in that** the control device has an inhibitor (235).

12. Implantable cardiac pacemaker according to claim 11, **characterised in that** the inhibitor (235) is co-operatively connected to an adaptation module (211) in such a way that adaptation is blocked as a function of the position.

13. Implantable cardiac pacemaker according to any one of claims 1 to 12, **characterised in that** the pacemaker is connected to a sensor for the physiological demand, in particular an impedance sensor for measuring the intracardial or transthoracal impedance, and comprises control means for adjusting a stimulation or heart rate as a function of a signal from the physiological demand sensor, wherein the change in the stimulation or heart rate can be permanently limited in such a way that the heart rate limitation is effected as a function of an output signal from the movement sensor (4).

**Revendications**

1. Stimulateur cardiaque implantable comportant un dispositif de commande (2) qui comprend un détecteur de position, qui est relié à un capteur de déplacement (4), **caractérisé en ce que** le détecteur de position comporte un dispositif de classification (23) pour identifier des déplacements de brève durée.

2. Stimulateur cardiaque implantable selon la revendication 1, **caractérisé en ce que** le dispositif de classification (23) comprend un compteur (238) réalisant un comptage sur une brève durée et un dispositif de commutation (239).

3. Stimulateur cardiaque implantable selon la revendication 1 ou 2, **caractérisé en ce que** le détecteur de position comporte un dispositif de mémoire (25).

4. Stimulateur cardiaque implantable selon la revendication 3, **caractérisé en ce que** le dispositif de mémoire (25) comporte un intégrateur (251).

5. Stimulateur cardiaque implantable selon la revendication 4, **caractérisé en ce que** le compteur (238) exécutant un comptage de brève durée et l'intégrateur (251) sont intégrés dans un compteur (231).

6. Stimulateur cardiaque implantable selon l'une des revendications 2 à 5, **caractérisé en ce qu'**à la fin du signal de déplacement, il compare un état de comptage à au moins une valeur de seuil pouvant être prédéterminée, et délivre un signal de changement de position.

7. Stimulateur cardiaque implantable selon l'une des revendications 1 à 6, **caractérisé en ce qu'**il est prévu un compteur de déroulement (234), à l'entrée duquel est appliqué le signal de changement de position et à la sortie duquel est délivré un signal de blocage.

8. Stimulateur cardiaque implantable selon la revendication 7, **caractérisé en ce qu'**il est prévu un dispositif d'effacement pour le signal de blocage et dont le déclencheur est relié à un capteur de déplacement (4).

9. Stimulateur cardiaque implantable selon l'une des revendications 1 à 8, **caractérisé en ce que** le dispositif de commande possède un limiteur de rythme cardiaque (236), qui dépend de la position.

10. Stimulateur cardiaque implantable selon la revendication 9, **caractérisé en ce que** le signal de changement de position et un signal de cadence cardiaque sont appliqués au limiteur de cadence cardiaque (236).

11. Stimulateur cardiaque implantable selon l'une des revendications 1 à 10, **caractérisé en ce que** le dispositif de commande comporte un inhibiteur (235).

12. Stimulateur cardiaque implantable selon la revendication 11, **caractérisé en ce que** l'inhibiteur (235) est relié de manière à coopérer avec un module d'adaptation (211) de telle sorte qu'une adaptation est bloquée en fonction de la position.

13. Stimulateur cardiaque implantable selon l'une des revendications 1 à 12, **caractérisé en ce que** le stimulateur cardiaque est relié à un capteur pour le besoin physiologique, notamment un capteur d'impédance pour la mesure de l'impédance intracardiaque ou transthoracique et comprend un dispositif de commande pour le réglage d'une cadence de stimulation ou d'une cadence cardiaque en fonction d'un signal qui provient du capteur concernant le besoin physiologique, la variation de la cadence de stimulation et de la cadence cardiaque pouvant être limitées de façon permanente de telle sorte que la limitation de la cadence cardiaque s'effectue en fonction d'un signal de sortie du capteur de limitation (4).

Fig. 1

71 {
**Constants:**
MaxMotCnt = 525;
OrthoThres= 8;
MaxOrthCnt= 90;
}

72 {
**Initialization on application startup:**
MotCnt = MaxMotCnt;
OrthoCnt = 0;
MFlag = 0;
OrthoFlag = 0;
ThresRate = (BSR+MSR)/2;
}

73 {
```
Algorithm:
if (MFlag := 0) {
  if (MotCnt < MaxMotCnt)
    MotCnt += 1;
  if (MotCnt < OrthoThres)
    OrthoCnt = MaxOrthoCnt;
  else
    OrthCnt = 0;
  OrthoFlag = 0;
}
else(
  if (MotCnt != 0)
    MotCnt -= 1;
  if (OrthoCnt != 0) {
    OrthoCnt -= 1;
    OrhtoFlag = 1;
  }
  else
    OrthoFlag = 0;
);

...
if (OrthoFlag != 0 )
  if (HRF > ThresRate)
    HRF = (HRF  ThresRate)/8 + ThresRate;

...
if (HRF > MSR) (
  if (OrthoFlag == 0 ) (
    Fac -= 1;

    ...
  );
);


...
// AVREF routine
if (OrthoFlag == 0)
  CETA = mREF;
else
  if (mREF > 1)
    CETA = mREF >> 1;
```
}

## Fig. 2

Fig. 3a

Fig. 3b

Fig. 3c

Fig. 3d

T$_{14}$ T$_{16}$ T$_{18}$

Fig. 4a

100

100

Fig. 4b

20

100

Fig. 4c

100

Fig. 4d

T$_{13}$                T$_{15}$  T$_{17}$

Fig. 5